# EUROPEAN PATENT SPECIFICATION

(11) **EP 0 945 132 B1**
(45) Date of publication and mention of the grant of the patent: **06.05.2004**
(21) Application number: 99300004.1
(22) Date of filing: 04.01.1999
(51) Int. Cl.: A61K 31/19, A61K 9/00, A61K 47/12

(54) **Ibuprofen composition**
Ibuprofenhaltige Arzneizubereitung
Composition d'ibuprofène

(30) Priority: 02.01.1998 US 2447
(43) Date of publication of application: 29.09.1999
(73) Proprietor: McNeil-PPC, Inc., Skillman, NJ 08558-9418 (US)
(72) Inventor: Bunick, Frank J., Randolph, NJ 07896 (US); Lin, Feng, Horsham, PA 19044 (US)
(74) Representative: Mercer, Christopher Paul

(56) References cited:
- EP-A- 0 181 564
- EP-A- 0 505 872
- EP-A- 0 753 296
- WO-A-91/16043
- WO-A-92/00731
- WO-A-97/02017
- US-A- 4 835 187
- US-A- 5 646 131

## Description

### BACKGROUND OF THE INVENTION

The present invention relates to a method of reducing the after taste burn of propionic acid compositions, more specifically ibuprofen compositions.

Many flavors and sweeteners have been added to medication in order to make them more palatable and to mask the burn and after taste which is common with many medications. Despite numerous efforts to find an effective means to eliminate this burn, there is a continuing need for a method to effectively eliminate the burning sensation with mediations, preferably the burn can be reduced to a level such that a chewable composition can be provided.

Ibuprofen is a well known medication which possesses an unpalatable burning feeling in the mouth and throat after ingestion.

EP-A-0 753 296 of American Home Products Corporation attempts to eliminate the unpalatable aftertaste by providing only one enantiomer. The patent application discloses the separation of ibuprofen from its racemic mixture to form an orally administered drug composition which contains only the S(+)-ibuprofen and essentially no R(-)-ibuprofen. While this approach may provide a more palatable form of ibuprofen the separation and isolation of the enantiomers are difficult.

US-A-4,762,702 discloses ibuprofen particles enveloped by a coating of hydro-colloid and fumaric acid. The preferred hydro-colloid composition comprises xantham gum and/or maltodextrin. The resulting product is an effervescent mixture which is vacuum dried. The patent discloses this method as overcoming the shortcoming of prior art preparations due to the envelopment of the ibuprofen crystals by the hydrocolloid in the presence of the fumaric acid.

Despite the disclosure of the above patents, a simpler and less costly method is providing a tastemasked ibuprofen composition.

### SUMMARY OF THE INVENTION

The present invention provides a method for reducing the after taste burn of a propionic acid derivative as defined in claims 1 to 4 of the appendant claims. The present invention provides fumaric acid sufficient to reduce the burn after-taste of propionic acid in the absence of a hydro-colloid agent. In a preferred embodiment the propionic acid derivative-fumaric composition so prepared is provided in a chewable form, but may also be a liquid formulation.

### DETAILED DESCRIPTION OF THE INVENTION

Propionic acid derivatives are a well known class of analgesic compounds. As used herein propionic acid derivatives are understood to include, but are not limited to, ibuprofen, naproxen benoxaprofen, naproxen sodium, flurbiprofen, fenoprofen, fenbuprofen, ketoprofen indoprofen, pirprofen, carpofen, oxaprofen, pranoprofen, microprofen, tioxaprofen, suproprofen, alminoprofen, tiaprofenic acid, fluprofen and bucloxic acid. The structural formula is set forth in US Patent 4,923,898. Propionic acid derivatives as defined herein are defined as pharmaceutically acceptable analgesics/non-steroidal anti-inflammatory drugs having a free -CH(CH₃)COOH or -CH₂CH₂COOH or a pharmaceutically acceptable salt group, such as -CH(CH₃)COO-Na+ or CH₂CH₂COO-Na+, which are typically attached directly or via a carbonyl functionality to an aromatic ring system.

Propionic acid derivatives are typically administered on a daily ranging from about 50 to about 2000 milligrams, preferably from about 100 to 1600 and most preferably from about 200 to about 1200 milligrams.

Ibuprofen is a widely used, well known non-steroidal anti-inflammatory known propionic acid derivative. Ibuprofen is chemically known as 2-(4-isobutylphenyl)propionic acid. As used herein ibuprofen is understood to include 2-(4-isobutylphenyl)-propionic acid as well as the pharmaceutically acceptable salts. Suitable ibuprofen salts include arginine, lysine, hystadine, as well as other salts described in US Patent No. 4,279,926, 4,873,231, 5,424,075,5,510,385.

Fumaric acid is a widely available pharmaceutically acceptable acid. The concentration of fumaric acid present to inhibit the burn of propionic acid derivative will vary on the amount of burn reduction desired. Generally the level of fumaric acid is from about 50 to about 150 weight percent of the propionic acid derivative dosage. Typically the level of fumaric acid is from about 60 to about 100 percent by weight of the level of propionic acid derivative and most preferably from about 70 to about 90 percent by weight of the level of propionic acid derivative dosage.

Contrary to the teaching of prior disclosures, the present invention does not require the incorporation of a hydrocolloid material in order to be effective. The present invention may be incorporated in the following embodiments.

The simplest and preferred embodiment is the incorporation of fumaric acid in a matrix, i.e., freely and randomly provided in a mixture. In this embodiment, the propionic acid derivative, preferably ibuprofen, and fumaric acid are provided in a matrix within the caplet, tablet or capsule form.

In another embodiment of the invention the ibuprofen and fumaric acid are provided in a granulation. Typically this involves the admixing of the propionic acid derivative, fumaric acid as well as sugars, binders, water and other ingredients together using equipment well known in the art.

For example, US Pat. Number 5,429,825 discloses rotomelt granulation methods. The mixture is then dried and milled. The milled product is then in suitable form to be compressed into a tablet. Preferably disentgrants are added to the admixture to aid the release of the active ingredients to the user. In a highly preferred embodiment the propionic acid, fumaric acid is provided such that a chewable tablet is available to those who have difficulty in swallowing a tablet.

In another embodiment of the present invention the fumaric acid/propionic acid is provided in the presence of a non-hydrocolloid binder. Preferably the non-hydrocolloid binder is a pharmaceutically acceptable wax or fat. Suitable waxes and fats include, glyceryl monosterate, hydrogenated tallow, myristyl alcohol, myristic acid, stearyl alcohol, substituted monoglycerides, substituted diglycerides, substituted triglycerides, beeswax, carnuaba wax, japan wax acetylate monoglycerides and the like. Combinations of two or more of the non-colloid binders may be used. Preferably the melting point of the non-colloidal binders of the invention have a melting temperature from about 30 to about 100 C, most preferably from about 40 to about 85 C.

The non-hydrocolloid binder is manufactured by first melting the wax or fat, and then admixing the ibuprofen and fumaric acid combination. The combination is then milled to the appropriate size and then compressed into tablets using techniques well known to those with skill in the art.

The formulation of the present invention may also contain pharmaceutically acceptable excipients, fillers, flavors, diluents, lubricants, disintegration agents, suspension agents, stabilizer, binders, colorants, carriers and the like.

For example suitable carriers include lactose, starch, disclaim phosphate, calcium sulfate, kaolin, mannitol and powered sugar. Typical binders include starch gelatin, sugars such as dextrose, mannitol, xylitol, sorbitol, malodextrins, fructose, sucrose, molasses, lactose; natural and synthetic gums, carboxymethylcellulose, methylcelullose, polyvinylpyrrolidone, polyethylene glycol, ethyl cellulose and waxes. Lubricants include boric acid, sodium benzoate, sodium acetate, sodium chloride, leucine, polyethylene glycol and the like. Typical disintegrates include, starch derived from wood, maize, potato, and rice, methylcellulose, magnesium silicates, aluminum silicates, sucrose, dextrose, malodextrose, agar, benoite, alginic acid, wood products, guar gum, citric pulp, sodium lauryl sulfate and the like.

The present invention may be provided in liquid form, e.g. an elixir, suspension, or syrup. The liquid formulations are prepared using manufacturing methods and pharmaceutically acceptable surfactants, dispersants and diluents known in the art. Preferably the present invention is provided in caplets, capsules, tablets and most preferably in a chewable form.

As used throughout this specification burn is understood to mean the aftertaste, commonly identified as metallic, noted when taking ibuprofen. This aftertaste is different than bitterness inasmuch as the addition of a sweetener is not effective in reducing the aftertaste.

Alternatively the ibuprofen, fumaric acid composition may be added at appropriate levels to beverages, food and other edible compositions which may be desired. It is also anticipated that the ibuprofen/fumaric acid compositions of the present invention may also be employed in veterinarian applications.

Without wishing to be bound by any theory the incorporation of fumaric acid to the propionic acid derivative reduces the characteristic after taste burn by acidifying the saliva sufficiently to maintain the protonated form of the propionic acid derivative. The protonated form of the propionic acid derivative has low solubility and hence has low irritation to the throat mucosa. Unlike other acidulates, fumaric acid dissolves slowly such that its sour taste is minimal in the mouth but sufficient to acidify the throat. Other pharmaceutically acceptable acids, such as citric, malic, tataric acids are much more soluble than fumaric acid. These and other acids impart an unacceptable sour taste in the mouth very quickly. The dissolution is so rapid that the sour taste is perceived well before the saliva is sufficiently acidified.

The invention will now be illustrated by, but is not intended to be limited to, the following examples. In these examples it is understood that unless noted otherwise, all parts are weight percent.

### Example 1

Chewable tablets containing 100 milligrams (mg) of ibuprofen were prepared with either 70 mg of fumaric acid or with no fumaric acid. The chewable tablets which contain no fumaric acid were used as a control. A taste panel of eighteen subjects were asked to chew two control tablets and score the throat burn on a scale of 1 to 9 (highest level). After one hour, tablets containing the ibuprofen/fumaric acid combination were chewed and the subjects were asked to score the results using the same 1-9 scale.

The subjects rated the control tablets as moderately high, as a 7 on a 9 point scale, whereas the tablets containing the ibuprofen/fumaric acid combination were rated as moderately low, a 3 or 4 on the 9 point scale.

### Example 2

The following formulation was found to be effective in eliminating after burn. ibuprofen 100 milligrams (mg) coloring 1.76 mg; microcrystalline cellulose 84 mg; sweetener 11 mg; second sweetener 4 mg; flavoring 2 mg; lubricant 6 mg; excipient 465 mg; fumaric acid 65 mg.

### Example 3

Two subjects were used to determine the effective level of fumaric acid for a 100 mg dosage of ibuprofen. The subjects rated the ibuprofen/fumaric acid composition with the following scale. Burn intensity: extremely high 9; very high 8; moderately high 7; slightly high 6; neither high or low 5; slightly low 4; moderately low 3; very low 2; extremely low 1; no bitterness or burn 0. The average score of the test results are reported below:

| | |
|---|---|
| 30 mg fumaric acid | 7 |
| 40 mg fumaric acid | 6 |
| 50 mg fumaric acid | 3 |
| 60 mg fumaric acid | 1 |

This example demonstrates that 50 mg fumaric acid by weight per 100 mg of ibuprofen was effective in reducing the burn after taste of ibuprofen.

## Claims

1. A method for reducing the after taste burn of a propionic acid derivative comprising:
admixing an analgesically effective amount of a racemic propionic acid derivative; and from 50 to to 150 weight percent of fumaric acid based upon the weight of the propionic acid derivative;
wherein the fumaric acid and the propionic acid derivative are admixed in the absence of a hydrocolloid.

2. The method of claim 1 wherein the propionic acid derivative is ibuprofen.

3. The method of claim 1 or claim 2 wherein the fumaric acid and the propionic acid derivative are admixed in a granulation process.

4. The method of claim 3 wherein the granulation process is conducted with a non-hydrocolloid binder.

5. A composition for oral administration obtainable by the method of any of claims 1 to 4 in the form of a chewable tablet.

6. A composition for oral administration obtainable by the method of any of claims 1 to 4 in the form of a liquid formulation.

## Patentansprüche

1. Verfahren zur Verringerung des brennenden Nachgeschmacks eines Propionsäurederivats, umfassend: Vermischen einer analgetisch effektiven Menge eines racemischen Propionsäurederivats und von 50 bis 150 Gewichts% von Fumarsäure, basierend auf dem Gewicht des Propionsäurederivats; wobei die Fumarsäure und das Propionsäurederivat in der Abwesenheit eines Hydrokolloids vermischt werden.

2. Verfahren nach Anspruch 1, wobei das Propionsäurederivat Ibuprofen ist.

3. Verfahren nach Anspruch 1 oder 2, wobei die Fumarsäure und das Propionsäurederivat in einem Granulierungsverfahren vermischt werden.

4. Verfahren nach Anspruch 3, wobei das Granulierungsverfahren mit einem nichthydrokolloidalen Bindemittel durchgeführt wird.

5. Zusammensetzung zur oralen Verabreichung, erhältlich durch das Verfahren nach einem der Ansprüche 1 bis 4, in Form einer Kautablette.

6. Zusammensetzung zur oralen Verabreichung, erhältlich durch das Verfahren nach einem der Ansprüche 1 bis 4, in Form einer flüssigen Formulierung.

## Revendications

1. Procédé de réduction de brûlure en arrière-goût d'un dérivé d'acide propionique comprenant : le mélange d'une quantité analgésiquement efficace d'un dérivé d'acide propionique racémique ; et de 50 à 150 pour cent en poids d'acide fumarique basé sur le poids du dérivé d'acide propionique ;
dans lequel l'acide fumarique et le dérivé d'acide propionique sont mélangés en l'absence d'un hydrocolloïde.

2. Procédé selon la revendication 1, dans lequel le dérivé d'acide propionique est l'ibuprofène.

3. Procédé selon la revendication 1 ou la revendication 2, dans lequel l'acide fumarique et le dérivé d'acide propionique sont mélangés dans un procédé de granulation.

4. Procédé selon la revendication 3, dans lequel le procédé de granulation est effectué avec un liant non hydrocolloïde.

5. Composition pour administration orale susceptible d'être obtenu par le procédé selon l'une quelconque des revendications 1 à 4 sous la forme d'un comprimé à mâcher.

6. Composition pour administration orale susceptible d'être obtenu par le procédé selon l'une quelconque des revendications 1 à 4 sous la forme d'une formulation liquide.
